(19) Europäisches Patentamt
European Patent Office
Office européen des brevets

(11) Veröffentlichungsnummer: **0 415 887 B1**

(12) # EUROPÄISCHE PATENTSCHRIFT

(45) Veröffentlichungstag der Patentschrift: **05.10.94**

(51) Int. Cl.[5]: **C07D 307/83**, C07D 407/04, C08K 5/15

(21) Anmeldenummer: **90810637.0**

(22) Anmeldetag: **22.08.90**

Die Akte enthält technische Angaben, die nach dem Eingang der Anmeldung eingereicht wurden und die nicht in dieser Patentschrift enthalten sind.

(54) **3-Phenylbenzofuran-2-one.**

(30) Priorität: **31.08.89 CH 3153/89**

(43) Veröffentlichungstag der Anmeldung:
**06.03.91 Patentblatt 91/10**

(45) Bekanntmachung des Hinweises auf die Patenterteilung:
**05.10.94 Patentblatt 94/40**

(84) Benannte Vertragsstaaten:
**BE DE ES FR GB IT NL SE**

(56) Entgegenhaltungen:
**EP-A- 0 034 749**
**WO-A-80/01566**

(73) Patentinhaber: **CIBA-GEIGY AG**
**Klybeckstrasse 141**
**CH-4002 Basel (CH)**

(72) Erfinder: **Dubs, Paul, Dr.**
**Route du Confin 14**
**CH-1723 Marly (CH)**
Erfinder: **Pitteloud, Rita, Dr.**
**Sur le Village**
**CH-1724 Praroman (CH)**

**EP 0 415 887 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue 3-Phenylbenzofuran2-one, das mit deren Hilfe gegen thermischen, oxidativen und aktinischen Abbau stabilisierte organische Material sowie die Verwendung dieser Verbindungen zum Stabilisieren von organischen Materialien.

Die Verwendung von einigen Benzofuran-2-onen als Stabilisatoren für organische Polymere ist aus der GB-A-2 042 562 oder der BE-A-881 496 sowie den Derwent Referaten 86-207 397/32, 86-214 695/33 und der Research Disclosure 288,28888 (1988) bekannt. In der US T 904003 werden einige Benzofuran-2-one und ihre Verwendung in der Farbphotographie beschrieben.

Die vorliegende Erfindung betrifft Verbindungen der Formel I,

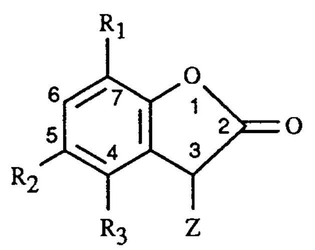

(I)

worin $R_1$ $C_{14}$-$C_{18}$-Alkyl bedeutet, $R_2$ Wasserstoff, $C_1$-$C_4$-Alkyl, $C_5$-$C_{12}$-Cycloalkyl, durch $C_1$-$C_4$-Alkyl substituiertes $C_5$-$C_7$-Cycloalkyl, Phenyl oder $C_7$-$C_{12}$-Phenylalkyl ist, $R_3$ Wasserstoff oder $C_1$-$C_4$-Alkyl bedeutet und Z Phenyl, durch $C_1$-$C_8$-Alkyl, $C_1$-$C_4$-Alkoxy oder Chlor substituiertes Phenyl, eine Gruppe

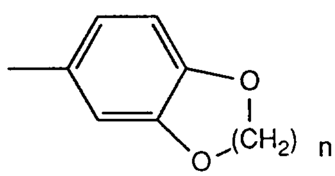

mit n gleich 1 oder 2 oder eine Gruppe

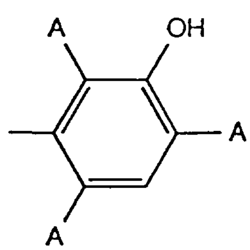

ist worin die Reste A unabhängig voneinander $C_1$-$C_8$-Alkyl, Methoxy oder Ethoxy bedeuten.

Beispiele für $R_1$ als $C_{14}$-$C_{18}$-Alkyl, sind Tetradecyl, Pentadecyl, Hexadecyl, Heptadecy und Octadecyl. Bevorzugt sind solche Alkylreste, die in 1-Stellung verzweigt sind, insbesondere 1-($C_{12}$-$C_{16}$-Alkyl)ethyl, zum Beispiel 1-Dodecylethyl, 1-Tridecylethyl, 1-Tetredecylethyl, 1-Pentadecylethyl und 1-Hexadecylethyl. Besonders bevorzugt sind 1-Dodecylethyl und 1-Hexadecylethyl.

Beispiele für $R_2$ als $C_1$-$C_4$-Alkyl sind Methyl, Ethyl, Propyl und Butyl. Eine bevorzugte Bedeutung von $R_2$ ist Methyl und tert-Butyl.

Beispiele für $R_2$ als $C_5$-$C_{12}$-Cycloalkyl sind Cyclopentyl, Cyclohexyl, Cycloheptyl, Cyclooctyl, Cyclononyl, Cyclodecyl, Cycloundecyl oder Cyclododecyl. $C_5$-$C_7$-Cycloalkyl, insbesondere Cyclohexyl, ist eine weitere bevorzugte Bedeutung von $R_2$.

Beispiele für $R_2$ als $C_5$-$C_7$-Cycloalkyl, welches durch $C_1$-$C_4$-Alkyl substituiert ist, sind Methylcyclohexyl und tert-Butylcyclohexyl.

Beispiele für $R_2$ als $C_7$-$C_{12}$-Phenylalkyl sind Benzyl und 2-Phenylethyl.

Beispiele für $R_3$ als $C_1$-$C_4$-Alkyl sind Methyl, Ethyl, Propyl und Butyl. Eine bevorzugte Bedeutung von $R_3$ ist Methyl.

Beispiele für Z als substituiertes Phenyl sind

o-, m- oder p-Methylphenyl, o-, m- oder p-Ethylphenyl, o-, m- oder p-Butylphenyl, o-, m- oder p-Methoxyphenyl, o-, m- oder p-Ethoxyphenyl, o-, m- oder p-Chlorphenyl und 2,4,6-Trimethyl-5-hydroxyphenyl.

Bevorzugt ist Z Phenyl.

Bevorzugt sind Verbindungen der Formel I, worin Z Phenyl ist.

Ebenfalls bevorzugt sind Verbindungen der Formel I, worin $R_2$ Wasserstoff, $C_1$-$C_4$-Alkyl, Cyclohexyl, Phenyl oder Benzyl bedeutet.

Weiterhin bevorzugt sind Verbindungen der Formel I, worin $R_2$ Wasserstoff oder $C_1$-$C_4$-Alkyl bedeutet.

$R_3$ bedeutet besonders bevorzugt Wasserstoff oder Methyl.

Von besonderem Interesse sind Verbindungen der Formel I, worin $R_2$ und $R_3$ unabhängig voneinander Wasserstoff oder $C_1$-$C_4$-Alkyl bedeuten.

Bevorzugte Beispiele für Verbindungen der Formel I sind:

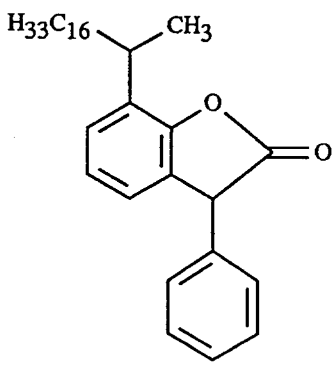 , 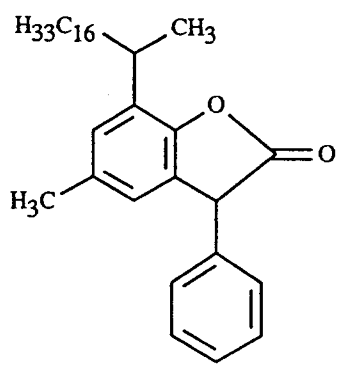 , 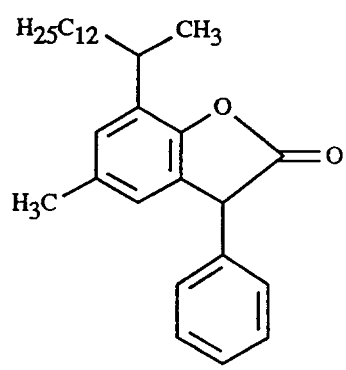 ,

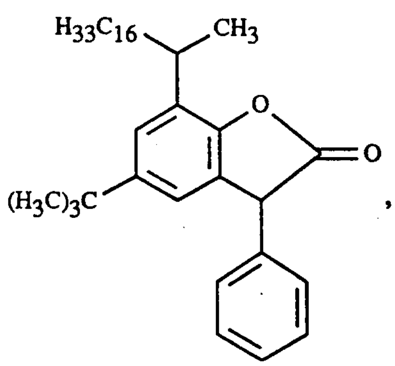 , 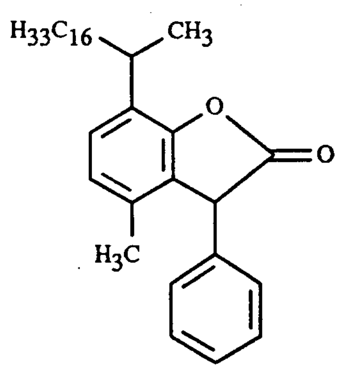 .

Eine besonders bevorzugte Verbindung der Formel I ist 3-Phenyl-5-methyl-7-(1'-dodecylethyl)benzofuran-2-on.

Die Verbindungen der Formel I eignen sich zum Stabilisieren von organischen Materialien gegen thermischen, oxidativen oder aktinischen Abbau.

Beispiele für derartige Materialien sind:

1. Polymere von Mono- und Diolefinen, beispielsweise Polypropylen, Polyisobutylen, Polybuten-1, Polymethylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE).

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).

3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere und LLDPE/Ethylen-Acrylsäure-Copolymere.
3a. Statistische oder alternierende Copolymere aus $\alpha$-Olefinen und Kohlenmonoxid.
3b. Kohlenwasserstoffharze (z.B. $C_5$-$C_9$) inklusive hydrierte Modifikationen davon (z.B. Klebrigmacherharze).
4. Polystyrol, Poly-(p-methylstyrol), Poly-($\alpha$-methylstyrol).
5. Copolymere von Styrol oder $\alpha$-Methylstyrol mit Dienen oder Acrylderivaten, wie z.B. Styrol-Butadien, Styrol-Acrylnitril, Styrol-Alkylmethacrylat, Styrol-Butadien-Alkylacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.
6. Pfropfcopolymere von Styrol oder $\alpha$-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.
7. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.
8. Polymere, die sich von $\alpha,\beta$-ungesättigten Säuren und deren Derivaten ableiten, wie Polyacrylate und Polymethacrylate, Polyacrylamide und Polyacrylnitrile.
9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.
10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.
11. Homo- und Copolymere von cyclischen Ethern, wie Polyalkylenglykole, Polyethylenoxyd, Polypropylenoxyd oder deren Copolymere mit Bisglycidylethern.
12. Polyacetale, wie Polyoxymethylen, sowie solche Polyoxymethylene, die Comonomere, wie z.B. Ethylenoxid, enthalten; Polyacetale, die mit termoplastischen Polyurethanen, Acrylaten oder MBS modifiziert sind.
13. Polyphenylenoxide und -sulfide und deren Mischungen mit Styrolpolymeren oder Polyamiden.
14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.
15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid,

Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten, wie z.B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z.B. von Bis-glycidylethern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.

27. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

Weitere Gegenstände der Erfindung sind daher Zusammensetzungen enthaltend ein gegen oxidativen, thermischen oder aktinischen Abbau empfindliches organisches Material und mindestens eine Verbindung der Formel I, sowie die Verwendung von Verbindungen der Formel I zum Stabilisieren von organischem Material gegen oxidativen, thermischen oder aktinischen Abbau.

Bevorzugte organische Materialien sind Polymere, z.B. synthetische Polymere oder thermoplastische Polymere. Besonders bevorzugte organische Materialien sind Polyolefine, z.B. Polypropylen oder Polyethylen.

Im allgemeinen werden die Verbindungen der Formel I dem zu stabilisierenden Material in Mengen von 0,01 bis 10 %, bevorzugt 0,01 bis 5 %, insbesondere 0,01 bis 2 %, bezogen auf das Gesamtgewicht des zu stabilisierenden Materials, zugesetzt.

Neben den Verbindungen der Formel I können die erfindungsgemässen Zusammensetzungen zusätzlich herkömmliche Additive enthalten, wie beispielsweise die unten angegebenen.

1. Antioxidantien

1.1. Alkylierte Monophenole, z.B. 2,6-Di-tert.butyl-4-methylphenol, 2-Tert.butyl-4,6-dimethylphenol, 2,6-Di-tert.butyl-4-ethylphenol, 2,6-Di-tert.butyl-4-n-butylphenol,2,6-Di-tert.butyl-4-i-butylphenol, 2,6-Dicyclopentyl-4-methylphenol, 2-($\alpha$-Methylcyclohexyl)-4,6-dimethylphenol, 2,6-Di-octadecyl-4-methylphenol, 2,4,6-Tri-cyclohexylphenol, 2,6-Di-tert.butyl-4-methoxymethylphenol, 2,6-Di-nonyl-4-methylphenol.

1.2. Alkylierte Hydrochinone, z.B. 2,6-Di-tert.butyl-4-methoxyphenol, 2,5-Di-tert.butylhydrochinon, 2,5-Di-tert.amyl-hydrochinon, 2,6-Diphenyl-4-octadecyloxyphenol.

1.3. Hydroxylierte Thiodiphenylether, z.B. 2,2'-Thio-bis-(6-tert.-butyl-4-methylphenol), 2,2'-Thio-bis-(4-octylphenol), 4,4'-Thio-bis-(6-tert.butyl-3-methylphenol), 4,4'-Thio-bis-(6-tert.butyl-2-methylphenol).

1.4. Alkyliden-Bisphenole, z.B. 2,2'-Methylen-bis-(6-tert.butyl-4-methylphenol), 2,2'-Methylen-bis-(6-tert.butyl-4-ethylphenol),2,2'-Methylen-bis-[4-methyl-6-($\alpha$-methylcyclohexyl)-phenol], 2,2'-Methylen-bis-(4-methyl-6-cyclohexylphenol), 2,2'-Methylen- bis-(6-nonyl-4-methylphenol), 2,2'-Methylen-bis-(4,6-di-tert.butylphenol), 2,2'-Ethyliden-bis-(4,6-di-tert.butylphenol), 2,2'-Ethyliden-bis-(6-tert.butyl-4-isobutylphenol), 2,2'-Methylen-bis-[6(α-methylbenzyl)-4-nonylphenol], 2,2'-Methylen-bis-[6-(α,α-dimethylbenzyl)-4-nonylphenol], 4,4'-Methylen-bis-(2,6-di-tert.-butylphenol), 4,4'-Methylen-bis-(6-tert.butyl-2-methylphenol), 1,1-Bis-(5-tert.butyl-4-hydroxy-2-methylphenyl)-butan, 2,6-Bis-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methyl-phenyl)-3-n-dodecylmercaptobutan, Ethylenglycol- bis-[3,3-bis-(3'-tert.-butyl-4'-hydroxyphenyl)-butyrat], Bis-(3-tert.butyl-4-hydroxy-5-methyl-phenyl)-dicyclopentadien, Bis-[2-(3'-tert.butyl-2'-hydroxy-5'-methyl-benzyl)-6-tert.butyl-4-methyl-phenyl]-terephthalat.

1.5 Benzylverbindungen, z.B. 1,3,5-Tris-(3,5-di-tert.butyl-4-hydroxy-benzyl)-2,4,6-trimethylbenzol, Bis-(3,5-di-tert.butyl-4-hydroxybenzyl)-sulfid, 3,5-Di-tert.butyl-4-hydroxybenzyl-mercaptoessigsäure-isooctylester, Bis-(4-tert.butyl-3-hydroxy-2,6-dimethylbenzyl)dithiol-terephthalat, 1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxybenzyl-phosphonsäure-dioctadecylester, Ca-Salz des 3,5-Di-tert.butyl-4-hydroxybenzyl-phosphonsäure-monoethylester, 1,3,5-Tris-(3,5-dicyclohexyl-4-hydroxybenzyl)isocyanurat.

1.6. Acylaminophenole, z.B. 4-Hydroxy-laurinsäureanilid, 4-Hydroxystearinsäureanilid, 2,4-Bis-(octylmercapto)-6-(3,5-di-tert.butyl-4-hydroxyanilino)-s-triazin, N-(3,5-di-tert.-butyl-4-hydroxyphenyl)-carbaminsäureoctylester.

1.7. Ester der β-(3,5-Di-tert.butyl-4-hydroxyphenyl)-propionsäure mit ein- oder mehrwertigen Alkoholen, wie z.B. mit Methanol, Octa- decanol, 1,6-Hexandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid.

1.8. Ester der β-(5-tert.Butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid.

1.9. Ester der β-(3,5-Dicyclohexyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid.

1.10. Amide der β-(3,5-Di-tert.butyl-4-hydroxyphenyl)-propionsäure, wie z.B. N,N'-Bis-(3,5-di-tert.butyl-4-hydroxyphenylpropionyl)-hexamethylendiamin, N,N'-Bis-(3,5-di-tert.butyl-4-hydroxyphenylpropionyl)-trimethylendiamin, N,N'-Bis-(3,5-di-tert.butyl-4-hydroxyphenylpropionyl)-hydrazin.

2. UV-Absorber und Lichtschutzmittel

2.1. 2-(2'-Hydroxyphenyl)-benztriazole, wie z.B. das 5'-Methyl-, 3',5'-Di-tert.butyl-, 5'-tert.Butyl-, 5'-(1,1,3,3-Tetramethylbutyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.-amyl-, 3',5'-Bis(α,α-dimethylbenzyl)-Derivat.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-tert.Butyl-phenylsalicylat, Phenylsalicylat, Octylphenyl-salicylat, Dibenzoylresorcin, Bis-(4-tert.butylbenzoyl)-resorcin, Benzoylresorcin, 3,5-Di-tert.butyl-4-hydroxybenzoesäure-2,4-di-tert.-butylphenylester, 3,5-Di-tert.butyl-4-hydroxybenzoesäurehexadecylester.

2.4. Acrylate, wie z.B. α-Cyan-β,β-diphenylacrylsäure-ethylester bzw. - isooctylester, α-Carbomethoxyzimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis-(2,2,6,6-tetramethyl-piperidyl)-sebacat, Bis-(1,2,2,6,6-pentamethylpiperidyl)-sebacat, n-Butyl-3,5-di-tert.butyl-4-hydroxybenzyl-malonsäure-bis(1,2, 2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert.Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon).

2.7. Oxalsäurediamide, wie z.B. 4,4'-Di-octyloxy-oxanilid, 2,2'-Di-octyloxy-5,5'-di-tert.-butyl-oxanilid, 2,2'-Di-dodecyloxy-5,5'di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyloxanilid und dessen Gemisch mit 2-Ethoxy-2'-ethyl-5,4'-di-tert.butyl-oxanilid, Gemische von o- und p-Methoxy- sowie von o- und p-Ethoxy-di-substituierten Oxaniliden.

2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin.

3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert.butyl-4-hydroxyphenylpropionyl)-hydrazi n, 3-Salicyloylamino-1,2,4-triazol, Bis-(benzyliden)-oxalsäuredihydrazid.

4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert.butylphenyl)-phosphit,Diisodecylpentaerythrit-diphosphit, Bis-(2,4-di-tert.butylphenyl)-pentaerythritdiphosphit, Tristearyl-sorbit-triphosphit, Tetrakis-(2,4-di-tert.butylphenyl)-4,4'-biphenylendiphosphonit, 3,9-Bis-(2,4-di-tert.butylphenoxy)-2,4,8,10-tetraoxa-3,9-diphosphaspiro[5.5]undecan.

5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z.B. 4-tert.Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z.B. Weichmacher, Gleitmittel, Emulgatoren, Pigmente, Optische Aufheller, Flammschutzmittel, Antistatika, Treibmittel.

Die herkömmlichen Additive werden beispielsweise in Konzentrationen von 0,01 bis 10 %, bezogen auf das Gesamtgewicht des zu stabilisierenden Materials, zugesetzt.

Die Einarbeitung der Verbindungen der Formel I sowie gegebenenfalls weiterer Additive in das polymere, organische Material erfolgt nach bekannten Methoden, beispielsweise vor oder während der Formgebung oder auch durch Aufbringen der gelösten oder dispergierten Verbindungen auf das polymere, organische Material, gegebenenfalls unter nachträglichem Verdunsten des Lösungsmittels. Die Verbindungen der Formel I können auch in Form eines Masterbatches, der diese beispielsweise in einer Konzentration von 2,5 bis 25 Gew.-% enthält, den zu stabilisierenden Materialien zugesetzt werden.

Die Verbindungen der Formel I können auch vor oder während der Polymerisation oder vor der Vernetzung zugegeben werden.

Die Verbindungen der Formel I können in reiner Form oder in Wachsen, Oelen oder Polymeren verkapselt in das zu stabilisierende Material eingearbeitet werden.

Die Verbindungen der Formel I können auch auf das zu stabilisierende Polymer aufgesprüht werden. Sie sind in der Lage, andere Zusätze (z.B. die oben angegebenen herkömmlichen Additive) bzw. deren Schmelzen zu verdünnen, so dass sie auch zusammen mit diesen Zusätzen auf das zu stabilisierende Polymer aufgesprüht werden können. Besonders vorteilhaft ist die Zugabe durch Aufsprühen während der Desaktivierung der Polymerisationskatalysatoren, wobei z.B. der zur Desaktivierung verwendete Dampf zum Versprühen verwendet werden kann.

Bei kugelförmig polymerisierten Polyolefinen kann es z.B. vorteilhaft sein, die Verbindungen der Formel I, gegebenenfalls zusammen mit anderen Additiven, durch Aufsprühen zu applizieren.

Eine bevorzugte Ausführungsform der vorliegenden Erfindung ist daher die Verwendung von Verbindungen der Formel I zum Stabilisieren von Polymeren gegen oxidativen, thermischen oder aktinischen Abbau, wobei die Verbindungen der Formel I auf das Polymer aufgesprüht werden.

Die so stabilisierten Materialien können in verschiedenster Form angewendet werden, z.B. als Folien, Fasern, Bändchen, Formmassen, Profile oder als Bindemittel für Lacke, Klebstoffe oder Kitte.

Die Verbindungen der Formel I können in Analogie zu bekannten Verfahren hergestellt werden; beispielsweise durch Kondensation von Phenolen der Formel II,

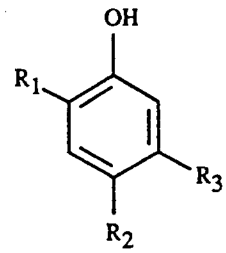

(II)

worin $R_1$ $R_2$ und $R_3$ die oben angegebene Bedeutung haben, mit 1-1,3 Mol Mandelsäure oder eines Mandelsäurederivats, wie in GB-A-2 042 562 beschrieben. An Stelle von Mandelsäure oder eines Mandelsäurederivats kann auch eine Verbindung der Formel III oder IV verwendet werden,

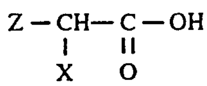

(III)

,

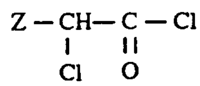

(IV)

wobei Z die oben angegebene Bedeutung besitzt und X z.B. Acetyloxy, Tolylsulfonyloxy oder Mesitylsulfonyloxy ist. Verwendet man eine Verbindung der Formel IV, so verfährt man zweckmässig wie in Beispiel 3 der BE-A-881 496 beschrieben.

Die Umsetzungen werden bevorzugt in einem Temperaturbereich von 180-220 °C und bei einem Druck von 10 - 1013 mbar durchgeführt.

Die Verbindungen der Formel II können, sofern sie nicht im Handel erhältlich sind, in Analogie zu bekannten Verfahren hergestellt werden; beispielsweise durch katalytische Alkylierung von Phenolen der Formel

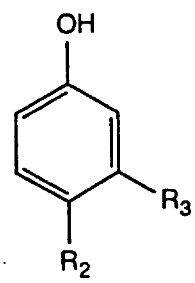

mit linearen $\alpha$-Olefinen ($C_{13}$-$C_{30}$). Alkyliert wird vorwiegend in flüssiger Phase ohne Verwendung eines Lösungsmittels.

Als geeignete Katalysatoren seien genannt:

a) Friedel-Crafts Katalysatoren wie z.B. Protonensäuren (Schwefelsäure, Phosphorsäure, p-Toluolsulfonsäure, etc.), anorganische Festkatalysatoren (säureaktivierende Bleicherden, z.B. Montmorillonit-Typen, Oxide des Aluminiums und Siliciums, etc.) und weitere Friedel-Crafts Katalysatoren (Bortrifluorid, Aluminiumchlorid, Eisen(III)-chlorid, Zinkchlorid, etc.),

b) o-selektive Katalysatoren wie z.B. Aluminiumphenolate und $\gamma$-Aluminiumoxid wie in US-A-3,367,981 und DE-B-1,142,873 beschrieben sowie andere Modifikationen des Aluminiumoxids.

Bevorzugt sind o-selektive Katalysatoren und besonders bevorzugt ist das in den oben angegebenen US-A und DE-B beschriebene aktive $\gamma$-Aluminiumoxid.

In Gegenwart von Friedel-Crafts Katalysatoren arbeitet man zweckmässigerweise unter "Normal-Druck" im Temperaturbereich von z.B. 30-150 °C. Bei der Alkylierung in Gegenwart von o-selektiven Katalysatoren ist es vorteilhaft, bei erhöhter Temperatur (ca. 200-350 °C) unter Druck (ca. 20-200 bar) zu arbeiten.

Nach der Alkylierung wird der Katalysator z.B. durch Auswaschen oder Filtration aus der Rohware entfernt, welche anschliessend destillativ gereinigt werden kann.

Die Verbindungen der Formel II können bei der Herstellung als Isomerengemisch anfallen, welches z.B. mit Hilfe von chromatographischen Methoden, insbesondere Gaschromatographie und Hochdruckflüssigchromatographie (HPLC), aufgetrennt werden kann. Es ist aber möglich, das Isomerengemisch bei der Herstellung der Verbindungen der

Formel I einzusetzen, so dass die Verbindungen der Formel I auch als Isomerengemisch anfallen. Dieses Isomerengemisch der Verbindungen der Formel I kann, falls erforderlich, aufgetrennt werden oder auch direkt zur Stabilisierung von organischen Materialien gegen oxidativen, thermischen oder aktinischen Abbau verwendet werden.

Für die Herstellung der Verbindungen der Formel II können auch handelsübliche Olefingemische ($H_2C=CH\text{-}CH_2\text{-}A_1$, worin $A_1$ z.B. $C_{13}$-$C_{17}$-Alkyl, $C_{17}$-$C_{21}$-Alkyl oder $C_{21}$-$C_{27}$-Alkyl bedeutet) eingesetzt werden.

Die folgenden Beispiele erläutern die Erfindung weiter. Teil- und Prozentangaben beziehen sich darin, soweit nichts anderes angegeben ist, auf das Gewicht.

Beispiel 1: Herstellung der Verbindung 3-Phenyl-7-(1'-hexadecylethyl)benzofuran-2-on.

4,8 g (31,5 mMol) Mandelsäure und 10,4 g (30 mMol) 1-Hexadecylethylphenol werden zusammen unter reduziertem Druck (13,3 mbar) während ca. 8 h auf 190-200 °C erhitzt. Dabei wird das bei der Reaktion entstehende Wasser abdestilliert. Nach Beendigung der Umsetzung wird das Reaktionsprodukt über Kieselgel (Hexan) filtriert und anschliessend mit kaltem Hexan behandelt. Das Produkt besitzt einen Schmelzpunkt von 60-62 °C und liegt als weisses Pulver vor. Die Ausbeute beträgt 7 g (= 50 % der Theorie).

| Elementaranalyse | | |
|---|---|---|
| Berechnet: | C 83,06 %; | H 10,02 % |
| Gefunden: | C 83,04 %; | H 10,02 % |

Beispiele 2-5: Die Herstellung der in Tabelle 1 angegebenen Verbindungen erfolgt in Analogie zu Beispiel 1.

Tabelle 1:

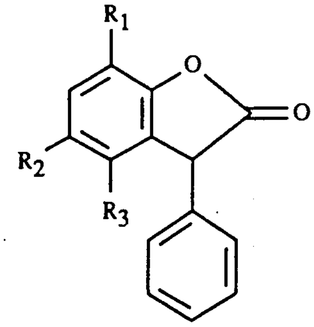

| Beispiel | $R_1$ | $R_2$ | $R_3$ | Smp. | Elementaranalyse |
|---|---|---|---|---|---|
| 2 | 1-Hexadecylethyl | -H | $-CH_3$ | Oel | ber.: C 83,14 %; H 10,15 %<br>gef.: C 83,11 %; H 10,33 % |
| 3 | 1-Hexadecylethyl | $-CH_3$ | -H | 42°C | ber.: C 83,14 %; H 10,15 %<br>gef.: C 83,43 %; H 10,33 % |
| 4 | 1-Dodecylethyl | $-CH_3$ | -H | Oel | ber.: C 82,81 %; H 9,59 %<br>gef.: C 82,88 %; H 9,76 % |
| 5 | 1-Hexadecylethyl | $-C(CH_3)_3$ | -H | Oel | ber.: C 83,34 %; H 10,49 %<br>gef.: C 83,47 %; H 10,47 % |

Beispiel 6: Stabilisierung Von Polypropylen.

1,3 kg Polypropylenpulver (Schmelzindex: 3,2 g/10 min, gemessen bei 230 °C/2,16 kg) werden mit 0,05 % Kalziumstearat, 0,05 % Tetrakis[3,5-di-tert-butyl-4-hydroxyphenylpropionyloxymethyl]methan und 0,05 % des in Tabelle 2 angegebenen Stabilisators vermischt. Diese Mischung wird in einem Extruder mit einem Zylinderdurchmesser von 20 mm und einer Länge von 400 mm bei 100 Umdrehungen pro Minute extrudiert, wobei die 3 Heizzonen auf 260 °C, 270 °C und 280 °C eingestellt sind. Das Extrudat wird zur Kühlung durch ein Wasserbad gezogen und anschliessend granuliert. Das erhaltene Granulat wird ein zweites und drittes Mal extrudiert. Nach diesen 3 Extrusionen wird der Schmelzindex bei 230 °C/2,16 kg gemessen. Die Ergebnisse sind in Tabelle 2 aufgeführt.

Tabelle 2

| Verbindung aus Beispiel Nr. | Schmelzindex*) [g/10 min] bei 230 ° C/2,16 kg |
|---|---|
| - | 17,2 |
| 1 | 4,6 |
| 2 | 5,9 |
| 3 | 4,5 |
| 4 | 4,3 |

*)Kleine Werte bedeuten eine gute Stabilisierung.

**Patentansprüche**

**Patentansprüche für folgende Vertragsstaaten : BE, DE, FR, GB, IT, NL, SE**

**1.** Verbindungen der Formel I,

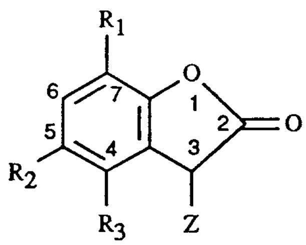

(I)

worin $R_1$ $C_{14}$-$C_{18}$-Alkyl bedeutet, $R_2$ Wasserstoff, $C_1$-$C_4$-Alkyl, $C_5$-$C_{12}$-Cycloalkyl, durch $C_1$-$C_4$-Alkyl substituiertes $C_5$-$C_7$-Cycloalkyl, Phenyl oder $C_7$-$C_{12}$-Phenylalkyl ist, $R_3$ Wasserstoff oder $C_1$-$C_4$-Alkyl bedeutet und Z Phenyl, durch $C_1$-$C_8$-Alkyl, $C_1$-$C_4$-Alkoxy oder Chlor substituiertes Phenyl, eine Gruppe

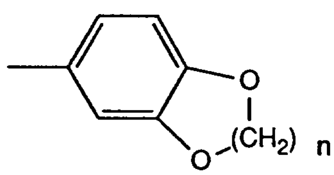

mit n gleich 1 oder 2 oder eine Gruppe

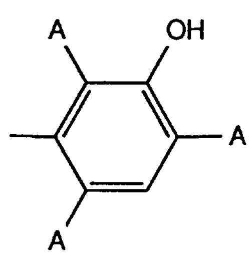

ist, worin die Reste A unabhängig voneinander $C_1$-$C_8$-Alkyl, Methoxy oder Ethoxy bedeuten.

**2.** Verbindungen gemäss Anspruch 1, worin Z Phenyl ist.

**3.** Verbindungen gemäss Anspruch 1, worin $R_2$ Wasserstoff, $C_1$-$C_4$-Alkyl, Cyclohexyl, Phenyl oder Benzyl bedeutet.

**4.** Verbindungen gemäss Anspruch 1, worin $R_2$ Wasserstoff oder $C_1$-$C_4$-Alkyl bedeutet.

**5.** Verbindungen gemäss Anspruch 1, worin $R_3$ Wasserstoff oder Methyl bedeutet.

**6.** Verbindungen gemäss Anspruch 1, worin $R_2$ und $R_3$ unabhängig voneinander Wasserstoff oder $C_1$-$C_4$-Alkyl bedeuten.

**7.** Die Verbindungen
3-Phenyl-7-(1'-hexadecylethyl)benzofuran-2-on,
3-Phenyl-5-methyl-7-(1'-hexadecylethyl)benzofuran-2-on,
3-Phenyl-5-methyl-7-(1'-dodecylethyl)benzofuran-2-on,
3-Phenyl-5-tert-butyl-7-(1'-hexadecylethyl)benzofuran-2-on und
3-Phenyl-4-methyl-7-(1'-hexadecylethyl)benzofuran-2-on gemäss Anspruch 1.

**8.** Die Verbindung 3-Phenyl-5-methyl-7-(1'-dodecylethyl)benzofuran-2-on gemäss Anspruch 1.

**9.** Zusammensetzung enthaltend ein gegen oxidativen, thermischen oder aktinischen Abbau empfindliches organisches Material und mindestens eine Verbindung gemäss Anspruch 1.

**10.** Verwendung von Verbindungen gemäss Anspruch 1 zum Stabilisieren eines organischen Materials gegen oxidativen, thermischen oder aktinischen Abbau.

**11.** Verwendung gemäss Anspruch 10, wobei das organische Material ein Polymer ist und die Verbindungen gemäss Anspruch 1 auf dieses Polymer aufgesprüht werden.

**Patentansprüche für folgenden Vertragsstaat : ES**

**1.** Zusammensetzung enthaltend ein gegen oxidativen, thermischen oder aktinischen Abbau empfindliches organisches Material und mindestens eine Verbindung der Formel I,

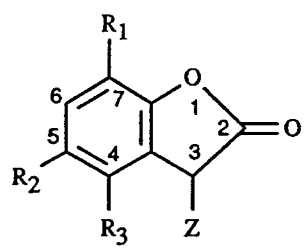

(I)

worin $R_1$ $C_{14}$-$C_{18}$-Alkyl bedeutet, $R_2$ Wasserstoff, $C_1$-$C_4$-Alkyl, $C_5$-$C_{12}$-Cycloalkyl, durch $C_1$-$C_4$-Alkyl substituiertes $C_5$-$C_7$-Cycloalkyl, Phenyl oder $C_7$-$C_{12}$-Phenylalkyl ist, $R_3$ Wasserstoff oder $C_1$-$C_4$-Alkyl bedeutet und Z Phenyl, durch $C_1$-$C_8$-Alkyl, $C_1$-$C_4$-Alkoxy oder Chlor substituiertes Phenyl, eine Gruppe

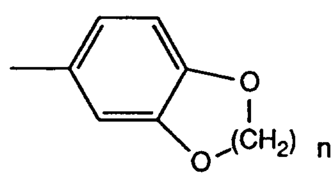

mit n gleich 1 oder 2 oder eine Gruppe

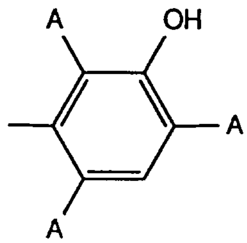

ist, worin die Reste A unabhängig voneinander $C_1$-$C_8$-Alkyl, Methoxy oder Ethoxy bedeuten.

2. Zusammensetzung gemäss Anspruch 1, worin Z Phenyl ist.

3. Zusammensetzung gemäss Anspruch 1, worin $R_2$ Wasserstoff, $C_1$-$C_4$-Alkyl, Cyclohexyl, Phenyl oder Benzyl bedeutet.

4. Zusammensetzung gemäss Anspruch 1, worin $R_2$ Wasserstoff oder $C_1$-$C_4$-Alkyl bedeutet.

5. Zusammensetzung gemäss Anspruch 1, worin $R_3$ Wasserstoff oder Methyl bedeutet.

6. Zusammensetzung gemäss Anspruch 1, worin $R_2$ und $R_3$ unabhängig voneinander Wasserstoff oder $C_1$-$C_4$-Alkyl bedeuten.

7. Zusammensetzung gemäss Anspruch 1, worin die Verbindung der Formel I
3-Phenyl-7-(1'-hexadecylethyl)benzofuran-2-on,
3-Phenyl-5-methyl-7-(1'-hexadecylethyl)benzofuran-2-on,
3-Phenyl-5-methyl-7-(1'-dodecylethyl)benzofuran-2-on,
3-Phenyl-5-tert-butyl-7-(1'-hexadecylethyl)benzofuran-2-on oder
3-Phenyl-4-methyl-7-(1'-hexadecylethyl)benzofuran-2-on ist.

8. Zusammensetzung gemäss Anspruch 1, worin die Verbindung der Formel I 3-Phenyl-5-methyl-7-(1'-dodecylethyl)benzofuran-2-on ist.

9. Verfahren zum Stabilisieren von organischem Material gegen oxidativen, thermischen oder aktinischen Abbau, dadurch gekennzeichnet, dass in das organische Material mindestens eine Verbindung der Formel I gemäss Anspruch 1 eingearbeitet wird.

10. Verfahren gemäss Anspruch 9, dadurch gekennzeichnet, dass das organische Material ein Polymer ist und die Verbindung der Formel I auf dieses Polymer aufgesprüht wird.

11. Verfahren zum Herstellen von Verbindungen der Formel I gemäss Anspruch 1, dadurch gekennzeichnet, dass ein Phenol der Formel II

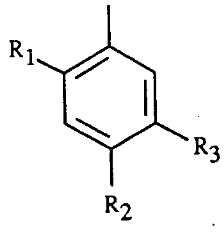

(II)

mit einer Verbindung der Formel

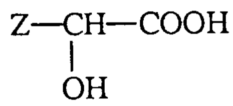

oder einer Verbindung der Formel III oder IV

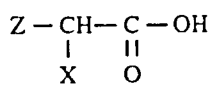

(III)

,

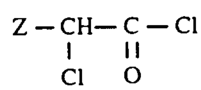

(IV)

umgesetzt wird, wobei $R_1$, $R_2$, $R_3$ und Z die in Anspruch 1 angegebenen Bedeutungen besitzen und X Acetyloxy, Tolylsulfonyloxy oder Mesitylsulfonyloxy ist.

**Claims**

**Claims for the following Contracting States : BE, DE, FR, GB, IT, NL, SE**

**1.** A compound of the formula I

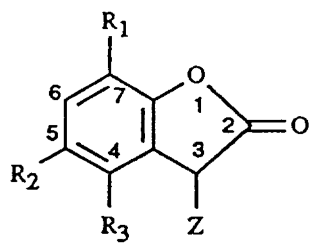

(I)

in which $R_1$ is $C_{14}$-$C_{18}$alkyl, $R_2$ is hydrogen, $C_1$-$C_4$alkyl, $C_5$-$C_{12}$cycloalkyl, $C_5$-$C_7$cycloalkyl which is substituted by $C_1$-$C_4$alkyl, or is phenyl or $C_7$-$C_{12}$phenylalkyl, $R_3$ is hydrogen or $C_1$-$C_4$alkyl and Z is phenyl, which is substituted by $C_1$-$C_8$alkyl, $C_1$-$C_4$alkoxy or chlorine, a group

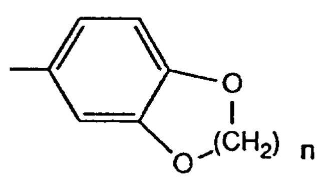

in which n is 1 or 2 or a group

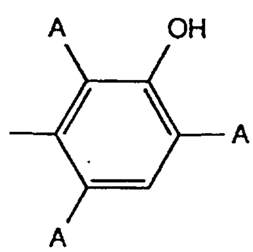

in which the radicals A independently of one another are $C_1$-$C_8$alkyl, methoxy or ethoxy.

2. A compound according to claim 1, wherein Z is phenyl.

3. A compound according to claim 1, wherein $R_2$ is hydrogen or $C_1$-$C_4$alkyl, phenyl or benzyl.

4. A compound according to claim 1, wherein $R_2$ is hydrogen or $C_1$-$C_4$alkyl.

5. A compound according to claim 1, wherein $R_3$ is hydrogen or methyl.

6. A compound according to claim 1, wherein $R_2$ and $R_3$ independently of one another are hydrogen or $C_1$-$C_4$alkyl.

7. The compound
3-phenyl-7-(1'-hexadecylethyl)benzofuran-2-one,
3-phenyl-5-methyl-7-(1'-hexadecylethyl)benzofuran-2-one,
3-phenyl-5-methyl-7-(1'-dodecylethyl)benzofuran-2-one,
3-phenyl-5-tert-butyl-7-(1'-hexadecylethyl)benzofuran-2-one or
3-phenyl-4-methyl-7-(1'-hexadecylethyl)benzofuran-2-one according to claim 1.

8. The compound
3-phenyl-5-methyl-7-(1'-dodecylethyl)benzofuran-2-one according to claim 1.

9. A composition containing an organic material which is sensitive to oxidative, thermal or actinic degradation, and at least one compound according to claim 1.

10. The use of a compound according to claim 1 for stabilizing an organic material against oxidative, thermal or actinic degradation.

11. The use according to claim 10, wherein the organic material is a polymer and the compound according to claim 1 is sprayed onto this polymer.

**Claims for the following Contracting State : ES**

1. A composition comprising an organic material sensitive to oxidative, thermal or actinic degradation and at least one compound of the formula I

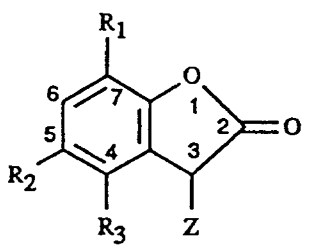

(I)

in which $R_1$ is $C_{14}$-$C_{18}$alkyl, $R_2$ is hydrogen, $C_1$-$C_4$alkyl, $C_5$-$C_{12}$cycloalkyl, $C_5$-$C_7$cycloalkyl which is substituted by $C_1$-$C_4$alkyl, or is phenyl or $C_7$-$C_{12}$phenylalkyl, $R_3$ is hydrogen or $C_1$-$C_4$alkyl and Z is phenyl, phenyl which is substituted by $C_1$-$C_8$alkyl, $C_1$-$C_4$alkoxy or chlorine, a group

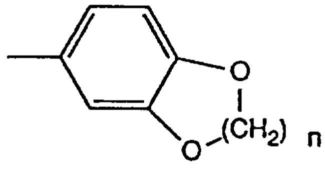

in which n is 1 or 2 or a group

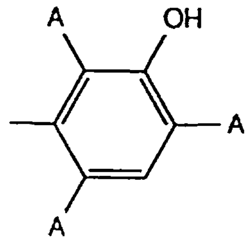

in which the radicals A independently of one another are $C_1$-$C_8$alkyl, methoxy or ethoxy.

**2.** A composition according to claim 1, wherein Z is phenyl.

**3.** A composition according to claim 1, wherein $R_2$ is hydrogen, $C_1$-$C_4$alkyl, cyclohexyl, phenyl or benzyl.

**4.** A composition according to claim 1, wherein $R_2$ is hydrogen or $C_1$-$C_4$alkyl.

**5.** A composition according to claim 1, wherein $R_3$ is hydrogen or methyl.

**6.** A composition according to claim 1, wherein $R_2$ and $R_3$ independently of one another are hydrogen or $C_1$-$C_4$alkyl.

**7.** A composition according to claim 1, in which the compound of the formula I is
3-phenyl-7-(1'-hexadecylethyl)benzofuran-2-one,
3-phenyl-5-methyl-7-(1'-hexadecylethyl)benzofuran-2-one,
3-phenyl-5-methyl-7-(1'-dodecylethyl)benzofuran-2-one,
3-phenyl-5-tert-butyl-7-(1'-hexadecylethyl)benzofuran-2-one or
3-phenyl-4-methyl-7-(1'-hexadecylethyl)benzofuran-2-one.

**8.** A composition according to claim 1, in which the compound of the formula I is
3-phenyl-5-methyl-7-(1'-dodecylethyl)benzofuran-2-one.

**9.** A process for stabilizing an organic material against oxidative, thermal or actiniic degradation, which comprises incorporating at least one compound of the formula I according to claim 1 into the organic material.

**10.** A process according to claim 9, wherein the organic material is a polymer and the compound of the formula I is sprayed onto this polymer.

**11.** A process for the preparation of a compound of the formula I according to claim 1, which comprises reacting a phenol of the formula II

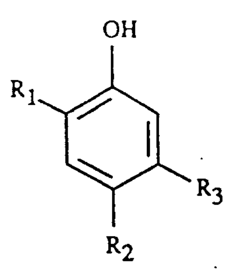

(II)

with a compound of the formula

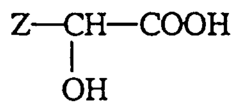

or with a compound of the formula III or IV

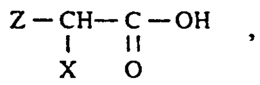

(III)

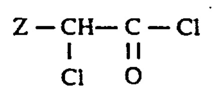

(IV)

in which $R_1$, $R_2$, $R_3$ and Z are as defined in claim 1 and X is acetyloxy, tolylsulfonyloxy or mesitylsulfonyloxy.

**Revendications**
**Revendications pour les Etats contractants suivants : BE, DE, FR, GB, IT, NL, SE**

**1.** Composés de formule I

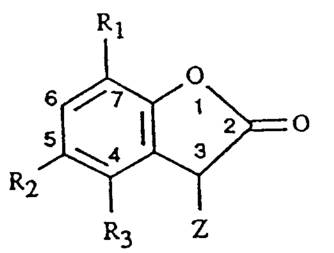

(I)

dans laquelle $R_1$ représente un alkyle en $C_{14}$-$C_{18}$, $R_2$ est un hydrogène, un alkyle en $C_1$-$C_4$, un cycloalkyle en $C_5$-$C_{12}$, un cycloalkyle en $C_5$-$C_7$ substitué par alkyle en $C_1$-$C_4$, un phényle ou un phénylalkyle en $C_7$-$C_{12}$, $R_3$ représente un hydrogène ou un alkyle en $C_1$-$C_4$ et Z est un phényle, un phényle substitué par alkyle en $C_1$-$C_8$, alcoxy en $C_1$-$C_4$ ou le chlore, un groupe

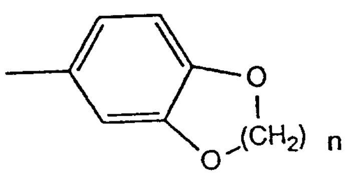

où n est égal à 1 ou 2, ou un groupe

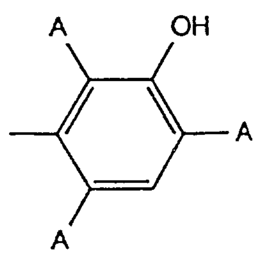

dans lequel les restes A représentent indépendamment les uns des autres un alkyle en $C_1$-$C_8$, un

méthoxy ou un éthoxy.

2. Composés selon la revendication 1, dans lesquels Z est un phényle.

3. Composés selon la revendication 1, dans lesquels $R_2$ représente un hydrogène, un alkyle en $C_1$-$C_4$, un cyclohexyle, un phényle ou un benzyle.

4. Composés selon la revendication 1, dans lesquels $R_2$ représente un hydrogène ou un alkyle en $C_1$-$C_4$.

5. Composés selon la revendication 1, dans lesquels $R_3$ représente un hydrogène ou un méthyle.

6. Composés selon la revendication 1, dans lesquels $R_2$ et $R_3$ représentent indépendamment l'un de l'autre un hydrogène ou un alkyle en $C_1$-$C_4$.

7. Composés selon la revendication 1, qui sont
la 3-phényl-7-(1'-hexadécyléthyl)benzofurane-2-one,
la 3-phényl-5-méthyl-7-(1'-hexadécyléthyl)benzofurane-2-one,
la 3-phényl-5-méthyl-7-(1'-dodécyléthyl)benzofurane-2-one,
la 3-phényl-5-tert-butyl-7-(1'-hexadécyléthyl)benzofurane-2-one,
la 3-phényl-4-méthyl-7-(1'-hexadécyléthyl)benzofurane-2-one.

8. Composé selon la revendication 1, qui est la 3-phényl-5-méthyl-7-(1'-dodécyléthyl)benzofurane-2-one.

9. Composition contenant une matière organique sensible à la dégradation due à l'oxydation, la chaleur ou la lumière, et au moins un composé selon la revendication 1.

10. Utilisation de composés selon la revendication 1 pour la stabilisation d'une matière organique contre la dégradation due à l'oxydation, la chaleur ou la lumière.

11. Utilisation selon la revendication 10, dans laquelle la matière organique est un polymère et les composés selon la revendication 1 sont pulvérisés sur ce polymère.

**Revendications pour l'Etat contractant suivant : ES**

1. Composition contenant une matière organique sensible à la dégradation due à l'oxydation, la chaleur ou la lumière, et au moins un composé de formule I

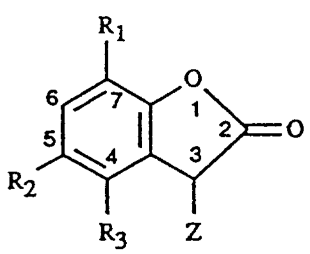

(I)

dans laquelle $R_1$ représente un alkyle en $C_{14}$-$C_{18}$, $R_2$ est un hydrogène, un alkyle en $C_1$-$C_4$, un cycloalkyle en $C_5$-$C_{12}$, un cycloalkyle en $C_5$-$C_7$ substitué par alkyle en $C_1$-$C_4$, un phényle ou un phénylalkyle en $C_7$-$C_{12}$, $R_3$ représente un hydrogène ou un alkyle en $C_1$-$C_4$ et Z est un phényle, un phényle substitué par alkyle en $C_1$-$C_8$, alcoxy en $C_1$-$C_4$ ou le chlore, un groupe

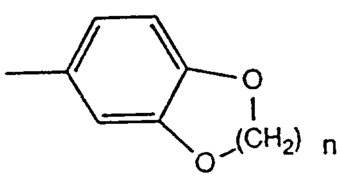

où n est égal à 1 ou 2, ou un groupe

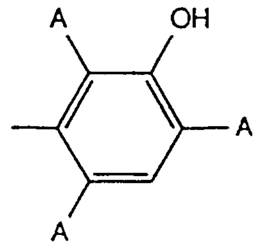

dans lequel les restes A représentent indépendamment les uns des autres un alkyle en $C_1$-$C_8$, un méthoxy ou un éthoxy.

**2.** Composition selon la revendication 1, dans laquelle Z est un phényle.

**3.** Composition selon la revendication 1, dans laquelle $R_2$ représente un hydrogène, un alkyle en $C_1$-$C_4$, un cyclohexyle, un phényle ou un benzyle.

**4.** Composition selon la revendication 1, dans laquelle $R_2$ représente un hydrogène ou un alkyle en $C_1$-$C_4$.

**5.** Composition selon la revendication 1, dans laquelle $R_3$ représente un hydrogène ou un méthyle.

**6.** Composition selon la revendication 1, dans laquelle $R_2$ et $R_3$ représentent indépendamment l'un de l'autre un hydrogène ou un alkyle en $C_1$-$C_4$.

**7.** Composition selon la revendication 1, dans laquelle le composé de formule I est
la 3-phényl-7-(1'-hexadécyléthyl)benzofurane-2-one,
la 3-phényl-5-méthyl-7-(1'-hexadécyléthyl)benzofurane-2-one,
la 3-phényl-5-méthyl-7-(1'-dodécyléthyl)benzofurane-2-one,
la 3-phényl-5-tert-butyl-7-(1'-hexadécyléthyl)benzofurane-2-one,
la 3-phényl-4-méthyl-7-(1'-hexadécyléthyl)benzofurane-2-one.

**8.** Composition selon la revendication 1, dans laquelle le composé de formule I est la 3-phényl-5-méthyl-7-(1'-dodécyléthyl)benzofurane-2-one.

**9.** Procédé de stabilisation d'une matière organique contre la dégradation due à l'oxydation, la chaleur ou la lumière, caractérisé en ce que l'on incorpore dans la matière organique au moins un composé de formule I selon la revendication 1.

**10.** Procédé selon la revendication 9, caractérisé en ce que la matière organique est un polymère et en ce que le composé de formule I est pulvérisé sur ce polymère.

**11.** Procédé de préparation de composés de formule I selon la revendication 1, caractérisé en ce que l'on fait réagir un phénol de formule II

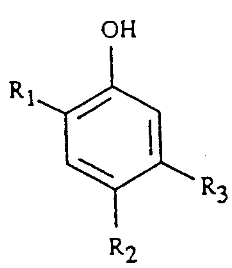

(II)

avec un composé de formule

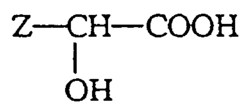

ou un composé de formule III ou IV

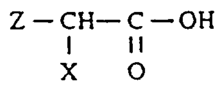

(III)

,

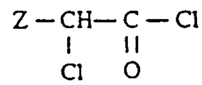

(IV)

où $R_1$, $R_2$, $R_3$ et Z ont les significations données dans la revendication 1, et X est un reste acétyloxy, tolylsulfonyloxy ou mésitylsulfonyloxy.